(19) Europäisches Patentamt
European Patent Office
Office européen des brevets

(11) EP 4 729 061 A1

(12) **EUROPEAN PATENT APPLICATION**
published in accordance with Art. 153(4) EPC

(43) Date of publication:
**22.04.2026 Bulletin 2026/17**

(21) Application number: **24822830.6**

(22) Date of filing: **14.06.2024**

(51) International Patent Classification (IPC):
***A61K 31/519** (2006.01)* ***A61K 45/06** (2006.01)*
***A61P 35/00** (2006.01)* ***A61P 35/04** (2006.01)*

(52) Cooperative Patent Classification (CPC):
**A61K 31/519; A61K 45/06; A61P 35/00; A61P 35/04**

(86) International application number:
**PCT/CN2024/099397**

(87) International publication number:
**WO 2024/255883 (19.12.2024 Gazette 2024/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.06.2023 CN 202310707237**

(71) Applicant: **Nanjing Chia Tai Tianqing Pharmaceutical Co., Ltd.**
**Nanjing, Jiangsu 210046 (CN)**

(72) Inventors:
- **JI, Xiaojun**
**Nanjing, Jiangsu 210046 (CN)**
- **ZHOU, Yumeng**
**Nanjing, Jiangsu 210046 (CN)**
- **ZHOU, Qiuhua**
**Nanjing, Jiangsu 210046 (CN)**
- **ZUO, Rui**
**Nanjing, Jiangsu 210046 (CN)**
- **PEI, Junjie**
**Nanjing, Jiangsu 210046 (CN)**
- **TAN, Xiaoyun**
**Nanjing, Jiangsu 210046 (CN)**
- **MIAO, Lei**
**Nanjing, Jiangsu 210046 (CN)**
- **DAI, Qingyu**
**Nanjing, Jiangsu 210046 (CN)**
- **MA, Changyou**
**Nanjing, Jiangsu 210046 (CN)**
- **WU, Jian**
**Nanjing, Jiangsu 210046 (CN)**
- **XU, Dan**
**Nanjing, Jiangsu 210046 (CN)**
- **ZHU, Chunxia**
**Nanjing, Jiangsu 210046 (CN)**

(74) Representative: **Elzaburu S.L.P.**
**Edificio Torre de Cristal**
**Paseo de la Castellana 259 C, planta 28**
**28046 Madrid (ES)**

# (54) USE OF AKT INHIBITOR IN PREPARATION OF DRUG FOR PREVENTING OR TREATING BREAST CANCER

(57) Disclosed in the present invention is a use of an AKT inhibitor in the preparation of a drug for preventing or treating breast cancer. In particular, provided is a use of an AKT inhibitor combined with fulvestrant in the preparation of a drug for preventing or treating breast cancer. The AKT inhibitor combined with the fulvestrant can effectively inhibit the growth of breast tumor cells and cell clone formation, has a significantly better effect than using either of the two alone, achieves an additive or synergistic effect, has low toxic and side effects on normal cells and high safety, provides a more effective method for treating breast cancer, and has an important clinical value.

EP 4 729 061 A1

## Description

The application claims the priority of the Chinese patent application No. 2023107072372 filed on June 14, 2023, and the entire text of the aforementioned Chinese patent application is incorporated herein by reference.

## TECHNICAL FIELD

The present disclosure belongs to the field of biomedicine, and specifically relates to a use of an AKT inhibitor in the manufacture of a medicament for preventing or treating breast cancer.

## BACKGROUND

Cancer is a disease with high mortality rate second only to cardiovascular diseases, and the number of cancer patients and deaths worldwide is constantly increasing. Among them, breast cancer is the most common malignant tumor globally, with an increasing incidence year by year, and it is the "number one killer" endangering women's lives. According to the driver genes of breast cancer, it can be divided into six subtypes, namely: PI3K/Akt/mTOR-driven subtype, growth factor receptor (ERBB2, EGFR, FGFR1)-driven subtype, cell cycle factor (CCND1, CDK4, RB)-driven subtype, ER-driven subtype, and BRCA1/2-driven subtype respectively. Studies have found that the PI3K-Akt signaling pathway plays an important role in 60% of breast cancers. Due to the high activation rate and crucial role of PI3K/Akt, multiple PI3K/Akt inhibitors have been approved for breast cancer indications or are undergoing clinical trials in various types of breast cancers.

Targeted therapy specifically targets the altered genes or proteins in tumor cells, featuring advantages of strong specificity, significant therapeutic effect, mild toxic and side effects, etc. It has become a brand-new treatment method beyond surgery, radiotherapy, and chemotherapy which are the three traditional therapeutic approaches, bringing benefits to a large number of breast cancer patients.

AKT is a class of serine/threonine kinases that affects cell survival, growth, metabolism, proliferation, migration, and differentiation through numerous downstream effectors. Aberrant activation of AKT is observed in more than 50% of human tumors. Aberrant activation of AKT can lead to tumorigenesis, metastasis, and drug resistance. AKT has three isoforms: AKT1, AKT2, and AKT3. As a typical protein kinase, each isoform consists of an amino-terminal PH domain (Pleckstrin homology domain), a central ATP-binding kinase domain, and a carboxyl-terminal regulatory domain. The three isoforms share approximately 80% amino acid sequence homology, with significant variations only in the linker region between the PH domain and the kinase domain. Therefore, inhibiting AKT activity can not only avoid the severe side effects caused by inhibiting upstream PI3K but also prevent the negative feedback mechanism induced by inhibiting downstream mTOR from affecting therapeutic efficacy. Molecular targeted therapy targeting AKT has gradually gained attention and become an important direction in the current development of anti-tumor targeted drugs.

WO2020156437A1 discloses a compound represented by Formula (A), the chemical name thereof is (R)-4-((1S,6R)-5-((S)-2-(4-chlorophenyl)-3-(isopropylamino)propanoyl)-2,5-diazabicyclo[4.1.0] heptan-2-yl)-5-methyl-5,8-dihydropyrido[2,3-d]pyrimidin-7(6H)-one. Its chemical structural formula is shown in the following Formula (A):

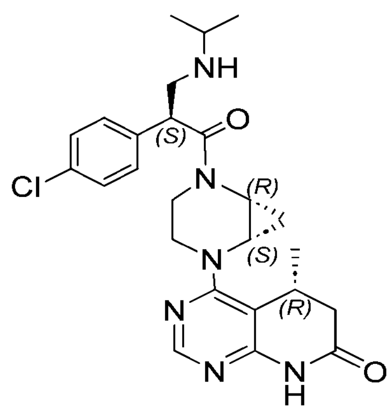

Formula (A)

The compound represented by Formula (A) is an AKT protein kinase inhibitor that can effectively inhibit AKT kinase activity and is used for the treatment of locally advanced or metastatic solid tumors. However, currently there are no

reports on the use of the compound represented by Formula (A) in combination with fulvestrant in the manufacture of a medicament for preventing or treating breast cancer.

## SUMMARY

The purpose of the present disclosure is to overcome the above-mentioned defects and shortcomings in the prior art, and to provide a use of an AKT inhibitor in the manufacture of a medicament for preventing or treating breast cancer; specifically, to provide a use of an AKT inhibitor combined with fulvestrant in the manufacture of a medicament for preventing or treating breast cancer.

To achieve the above purpose, the present disclosure provides the following technical solutions:
A use of an AKT inhibitor in the manufacture of a medicament for preventing or treating breast cancer, wherein, the AKT inhibitor is a compound represented by Formula (I) (hereinafter collectively referred to as the compound of Formula (I)), or a pharmaceutically acceptable salt thereof, or a hydrate thereof.

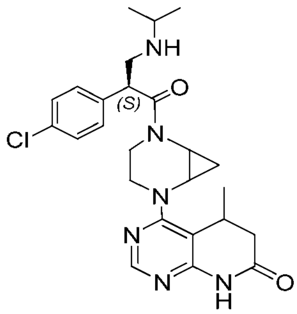

Formula (I).

In some embodiments, the compound of Formula (I) is the compound of Formula (A) or the compound of Formula (B):

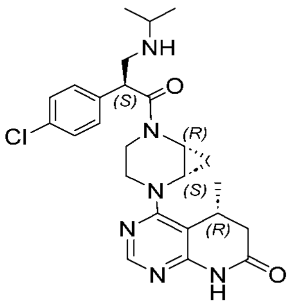 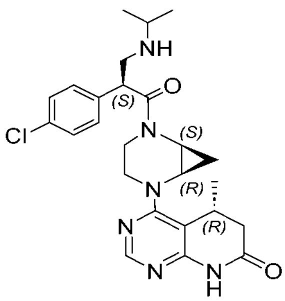

Formula (A) Formula (B).

The present disclosure also provides a use of an AKT inhibitor in the manufacture of a medicament for preventing or treating breast cancer, wherein the AKT inhibitor is the compound represented by Formula (A) (hereinafter collectively referred to as the compound of Formula (A)), or pharmaceutically acceptable salt thereof, or hydrate thereof.

In preferred embodiments of the present disclosure, the breast cancer is hormone receptor positive breast cancer.

In preferred embodiments of the present disclosure, the breast cancer is HR positive breast cancer.

In preferred embodiments of the present disclosure, the breast cancer is HR positive and HER-2 negative breast cancer.

In preferred embodiments of the present disclosure, the breast cancer is ER positive, PR positive and HER-2 negative hormone receptor positive breast cancer.

In preferred embodiments of the present disclosure, the breast cancer is HR positive and HER-2 negative breast cancer which is refractory to standard treatment, locally advanced or metastatic.

In preferred embodiments of the present disclosure, the breast cancer is HR positive and HER-2 negative breast cancer which has or does not have PI3K/Akt/PTEN pathway alteration, and is refractory to standard treatment, locally advanced or metastatic.

In preferred embodiments of the present disclosure, the breast cancer is HR positive and HER-2 negative breast

cancer which has PI3K/Akt/PTEN pathway alteration, and is refractory to standard treatment, locally advanced or metastatic.

In preferred embodiments of the present disclosure, the breast cancer is HR positive and HER-2 negative breast cancer which does not have PI3K/Akt/PTEN pathway alteration, and is refractory to standard treatment, locally advanced or metastatic.

In preferred embodiments of the present disclosure, the compound of Formula (A) is used in combination with fulvestrant.

In one embodiment of the present disclosure, the administration mode of the combined use is selected from simultaneous, concurrent, independent or sequential application.

In one embodiment of the present disclosure, the administration route of the combined use is selected from modes of oral, parenteral, rectal, pulmonary, topical administration, etc., wherein the parenteral administration includes but is not limited to intravenous injection, subcutaneous injection, and intramuscular injection.

In one embodiment of the present disclosure, the administration dose of the compound of Formula (A) is 0.1-1000 mg, and specifically the dose is optionally selected from 1 mg, 2.5 mg, 5 mg, 7.5 mg, 10 mg, 12.5 mg, 15 mg, 17.5 mg, 20 mg, 22.5 mg, 25 mg, 27.5 mg, 30 mg, 32.5 mg, 35 mg, 37.5 mg, 40 mg, 42.5 mg, 45 mg, 47.5 mg, 50 mg, 52.5 mg, 55 mg, 60 mg, 65 mg, 70 mg, 75 mg, 80 mg, 85 mg, 90 mg, 95 mg, 100 mg, 105 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 350 mg, 400 mg, 450 mg, 500 mg, 550 mg, 600 mg, 700 mg, 750 mg, 800 mg, 900 mg, or 1000 mg, as well as any value between any two of the above values (which, although not listed one by one, are deemed to be clearly indicated); preferably 20-500 mg; more preferably 100, 200, 300, 400, or 500 mg; further preferably 300, 400, or 500 mg.

In one embodiment of the present disclosure, the administration frequency of the compound of Formula (A) can be once a day, twice a day, three times a day, once a week, once every two weeks, once every three weeks, once a month, 21+7 (28 days as one cycle, once a day or twice a day for the first 21 days, followed by 7 days of rest), 4+3 (7 days as one cycle, once a day or twice a day for the first 4 days, followed by 3 days of rest), or 5+2 (7 days as one cycle, once a day or twice a day for the first 5 days, followed by 2 days of rest); preferably once a day, twice a day, 21+7 (28 days as one cycle, once a day or twice a day for the first 21 days, followed by 7 days of rest), 4+3 (7 days as one cycle, once a day or twice a day for the first 4 days, followed by 3 days of rest), or 5+2 (7 days as one cycle: once a day or twice a day for the first 5 days, followed by 2 days of rest).

In one embodiment of the present disclosure, the administration frequency of the compound of Formula (A) is once a day, twice a day, three times a day, once a week, once every two weeks, once every three weeks, or once a month; preferably once a day or twice a day.

In one embodiment of the present disclosure, the administration frequency of the compound of Formula (A) is once a day, twice a day, or three times a day, with continuous administration for 21 days followed by an interval of 1 to 2 weeks without administration of the compound; or continuous administration for 5 days or 4 days followed by an interval of 1 to 3 days without administration of the compound.

In one embodiment of the present disclosure, the administration frequency of the compound of Formula (A) is once a day or twice a day, with continuous administration for 21 days followed by an interval of 1 week without administration of the compound; or continuous administration for 5 days followed by an interval of 2 days without administration of the compound; or continuous administration for 4 days followed by an interval of 3 days without administration of the compound.

In one embodiment of the present disclosure, the administration dose of fulvestrant is 0.1-800 mg, and specifically the dose is optionally selected from 1 mg, 2.5 mg, 5 mg, 7.5 mg, 10 mg, 12.5 mg, 15 mg, 17.5 mg, 20 mg, 22.5 mg, 25 mg, 27.5 mg, 30 mg, 32.5 mg, 35 mg, 37.5 mg, 40 mg, 42.5 mg, 45 mg, 47.5 mg, 50 mg, 52.5 mg, 55 mg, 60 mg, 65 mg, 70 mg, 75 mg, 80 mg, 85 mg, 90 mg, 95 mg, 100 mg, 105 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 350 mg, 400 mg, 450 mg, 500 mg, 600 mg, 700 mg, 750 mg, or 800 mg, as well as any value between any two of the above values (which, although not listed one by one, are deemed to be clearly indicated); preferably the administration dose is 20-500 mg; more preferably 100, 200, 300 mg, 400 mg, or 500 mg; further preferably 300, 400, or 500 mg.

In one embodiment of the present disclosure, the administration frequency of fulvestrant can be once a day, once a week, once every two weeks, once every three weeks, once every four weeks, once a month, or once every two months; preferably once every two weeks or once every four weeks.

In one embodiment of the present disclosure, the administration frequency of the compound of Formula (A) is 21+7 (28 days as one cycle, once a day or twice a day for the first 21 days, followed by 7 days of rest); the administration frequency of fulvestrant is one cycle of 28 days, with administration once two weeks after the first administration in the first cycle, and then once on the first day of each subsequent cycle.

In one embodiment of the present disclosure, one cycle is 28 days; the administration frequency of the compound of Formula (A) is continuous administration for 21 days, followed by an interval of 1 week between each administration cycle; the administration frequency of fulvestrant is once two weeks after the first administration in the first cycle, and then

once on the first day of each subsequent cycle.

In one embodiment of the present disclosure, in terms of mass ratio, the dose ratio of the compound of Formula (A) to fulvestrant is selected from 0.001-500:1, preferably 0.01:1, 0.1:1, 0.2:1, 0.5:1, 1:1, 1.5:1, 2:1, 3:1, 5:1, 8:1, 10:1, 12:1, 15:1, 18:1, 20:1, 30:1, 40:1, 50:1, 100:1, 130:1, 150:1, 180:1, 200:1, 220:1, 250:1, 300:1, 350:1, 400:1, or 500:1, as well as any value between any two of the above values (which, although not listed one by one, are deemed to be clearly indicated); preferably 0.001-100:1.

In one embodiment of the present disclosure, the compound of Formula (A) and fulvestrant can be used in combined use alone, and can also be used in combination with other therapeutic agents or therapeutic methods for cancer treatment, such as combination therapy with other therapeutic agents including chemotherapy, hormones, antibodies, targeted drugs, etc., as well as surgery and/or radiotherapy.

In one embodiment of the present disclosure, the compound of Formula (A) can be administered to patients in the form of free bases, or can be administered in the form of pharmaceutically acceptable salts, hydrates, and prodrugs (which will be converted into the free base form in *vivo),* such as the fumarate form. The compound of Formula (A) or salts thereof can be prepared with reference to the preparation method disclosed in WO2022017448A1.

In one embodiment of the present disclosure, the pharmaceutically acceptable salt of the compound of Formula (A) is a monofumarate.

In one embodiment of the present disclosure, the pharmaceutically acceptable salt of the compound of Formula (A) is a monofumarate dihydrate, the chemical name is (R)-4-((1S,6R)-5-((S)-2-(4-chlorophenyl)-3-(isopropylamino) propanoyl)-2,5-diazabicyclo[4.1.0] heptan-2-yl)-5-methyl-5,8-dihydropyrido[2,3-d]pyrimidin-7(6H)-one fumarate dihydrate, and its chemical structral formula is shown in the following Formula (IA):

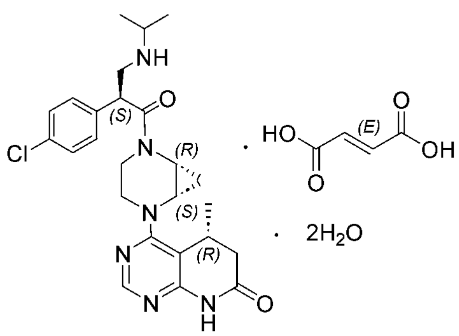

Formula (IA) .

The present disclosure also provides a pharmaceutical combination or pharmaceutical composition, which comprises the compound of Formula (I) or pharmaceutically acceptable salt thereof, and fulvestrant, as well as one or more pharmaceutically acceptable carriers;

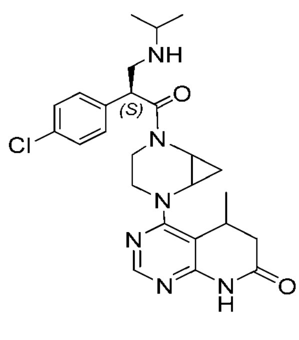

Formula (I) .

In some embodiments, the compound of Formula (I) is the compound of Formula (A) or the compound of Formula (B):

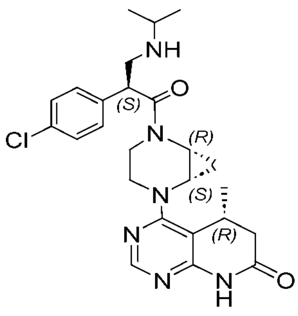 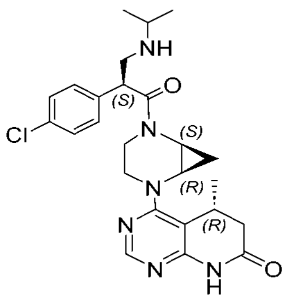

Formula (A) Formula (B) .

The present disclosure also provides a pharmaceutical combination, which comprises the compound of Formula (A) or pharmaceutically acceptable salt thereof, and fulvestrant, as well as one or more pharmaceutically acceptable carriers.

In the pharmaceutical combination or pharmaceutical composition, the administration mode, administration dose, administration frequency, and ratio of the compound of Formula (A) and/or fulvestrant are consistent with those in the aforementioned combined use part.

The present disclosure also provides a pharmaceutical composition, which comprises the compound of Formula (A) or pharmaceutically acceptable salt thereof, and fulvestrant, as well as one or more pharmaceutically acceptable carriers.

The pharmaceutical composition of the compound of Formula (A) and fulvestrant of the present disclosure can be formulated into any pharmaceutically acceptable dosage form. For example, it can be formulated into tablets, capsules, pills, granules, solutions, suspensions, syrups, injections (including injection solutions, sterile powders for injection, and concentrated solutions for injection), suppositories, inhalants, or sprays.

The pharmaceutical composition of the compound of Formula (A) and fulvestrant of the present disclosure can also be administered to patients or subjects in need of such treatment by any suitable administration route, such as oral, parenteral, rectal, pulmonary, or topical administration. When used for oral administration, the pharmaceutical composition can be formulated into oral preparations, for example, oral solid preparations such as tablets, capsules, pills, granules, etc.; or oral liquid preparations such as oral solutions, oral suspensions, syrups, etc. When formulated into oral preparations, the pharmaceutical preparations can also comprise suitable fillers, binders, disintegrants, lubricants, etc.

The pharmaceutical composition of the compound of Formula (A) and fulvestrant of the present disclosure can be administered alone, or can also be used in combination with other therapeutic agents or therapeutic methods for breast cancer treatment except fulvestrant, such as combination therapy with other therapeutic agents including chemotherapy, hormones, antibodies, targeted drugs, etc., as well as surgery and/or radiotherapy.

The present disclosure also provides a method for preventing and/or treating breast cancer, which comprises administering a therapeutically effective amount of the compound of Formula (A) and fulvestrant to a patient.

The present disclosure also provides a method for preventing and/or treating breast cancer, which comprises administering a therapeutically effective amount of the pharmaceutical composition of the compound of Formula (A) and fulvestrant to a patient.

**Beneficial Effects:**

The present disclosure provides a use of an AKT inhibitor in the manufacture of a medicament for preventing or treating breast cancer. The AKT inhibitor combined with the fulvestrant can effectively inhibit the growth of breast tumor cells and cell clone formation, has a significantly better effect than using either of the two alone, achieves an additive or synergistic effect, has low toxic and side effects on normal cells and high safety, provides a more effective method for treating breast cancer, and has important clinical value.

**Definition**

Unless otherwise specified, the following terms used in the description and claims have the following meanings: The "combination" herein refers to an administration mode. It comprises various scenarios of administering the two drugs sequentially or simultaneously, meaning that at least one dose of the compound of Formula (A) and at least one dose of fulvestrant are administered in any manner within a certain time period. The time period can be within one administration cycle, during which the compound of Formula (A) and fulvestrant can be administered simultaneously or sequentially; the any manner includes administering the compound of Formula (A) and fulvestrant *via* the same administration route or

different administration routes. For example, in one embodiment, one or more doses of the compound of Formula (A) are administered simultaneously or separately with one or more doses of fulvestrant; in one embodiment, multiple doses of the compound of Formula (A) are administered simultaneously or separately with multiple doses of fulvestrant; in one embodiment, multiple doses of the compound of Formula (A) are administered simultaneously or separately with one dose of fulvestrant; in one embodiment, one dose of the compound of Formula (A) is administered simultaneously or separately with multiple doses of fulvestrant; in one embodiment, one dose of the compound of Formula (A) is administered simultaneously or separately with one dose of fulvestrant; in all embodiments, the compound of Formula (A) can be administered first, or fulvestrant can be administered first. The term "simultaneous administration" refers to the administration of two or more drugs to a patient at the same time or within a very close time frame. The term "concurrent administration" refers to the administration of two or more drugs within the same period of time, such as on the same day, but not necessarily at the same time; the term "independent administration" refers to the administration of one drug independently within a period of time, such as the independent administration of one drug over the course of several days or a week, followed by the independent administration of other drugs within a subsequent period of time; the term "sequential administration" refers to the administration of drugs to a patient in a specific order, with each drug administered after the previous one, forming an ordered sequence.

The "oral administration" herein refers to ingesting drugs into the body through the oral route, where the drugs are absorbed through the gastrointestinal tract into the bloodstream, then distributed throughout the body or act on specific organs.

The "parenteral administration" herein refers to an administration route that does not involve the gastrointestinal tract, including subcutaneous injection, intramuscular injection, intravenous injection, etc.

The "rectal administration" herein refers to a way of delivering drugs into the rectum through the anus, where the drugs are absorbed into the bloodstream through the rectal mucosa.

The "pulmonary administration" herein refers to a way of directly delivering drugs into the lungs through inhalation, where the drugs are absorbed into the bloodstream through the alveoli.

The "topical administration" herein refers to an administration method where drugs are directly applied or spread on epithelial tissues, including the skin or mucous membranes of the oral cavity or vagina.

The "combined use" or "used in combination with..." herein is not intended to imply that the therapies or therapeutic agents must be physically mixed, administered simultaneously, and/or formulated for co-delivery, although these delivery methods are within the scope herein. The therapeutic agents in these combinations can be administered simultaneously with, prior to, or subsequent to one or more other additional therapies or therapeutic agents. The therapeutic agents can be administered in any order. In general, each therapeutic agent will be administered at the dosage and/or schedule determined for that particular agent. It should also be understood that the additional therapeutic agents used in the combination may be administered together in a single composition or separately in different compositions. In general, it is expected that the additional therapeutic agents used in the combination will be administered at levels not exceeding those when they are used alone. In one embodiment, the levels used in the combination will be lower than those used in monotherapy.

The "combined use", "combination use" herein can involve the joint use of the compound of Formula (A) or pharmaceutically acceptable salt thereof, and fulvestrant. Depending on the intended administration dose and frequency, the two compounds can be administered together or separately. The dosage and frequency of administration can vary depending on the compounds used and the specific condition to be treated. In general, it is preferable to use the minimum dosage sufficient to provide effective treatment, which can be determined by the following criteria, such as the patient's age, weight, and gender; the extent and severity of the cancer to be treated; and the judgment of the treating physician. The therapeutic effect in patients can usually be monitored using assays suitable for the condition being treated, and the optimal dosage can also be determined using routine tests and procedures well known in the art. For example, a single bolus injection can be administered, several divided doses can be given over time, or the dosage may be proportionally reduced or increased, as indicated by the urgency of the treatment situation. These assays are well known to those of ordinary skill in the art. In addition, it should be understood that the therapeutic agents of the combination of the present disclosure can be administered *via* any suitable route, which can vary depending on, for example, the health status of the recipient of the combination and the cancer to be treated.

The "pharmaceutical composition" herein refers to a mixture comprising at least one therapeutic agent and at least one pharmaceutically acceptable carrier. The "pharmaceutically acceptable carrier" includes but is not limited to diluents, binders, disintegrants, and lubricants.

The "administration dose" herein refers to the amount of the therapeutic agent. For example, when a 2 mg dose of the compound of Formula (A) is administered and the compound of Formula (A) is given in a tablet containing the compound of Formula (A) fumarate, the tablet will comprise an amount of the compound of Formula (A) fumarate equivalent to 2 mg of the compound of Formula (A).

The "salt" herein can exist alone or as a mixture with the free compound of the present disclosure, and is preferably a pharmaceutically acceptable salt. The "pharmaceutically acceptable salt" refers to commonly used organic

acid salts or inorganic acid salts in pharmacy, wherein the organic acid salts include but are not limited to fumarate, mesylate, isethionate, α-naphthalenesulfonate, p-toluenesulfonate, 1,2-ethanedisulfonate, oxalate, maleate, citrate, succinate, L-(+)-tartrate, hippurate, L-ascorbate, L-malate, benzoate, or gentisate; the inorganic acid salts include but are not limited to hydrochloride, sulfate, or phosphate. In one embodiment, the "pharmaceutically acceptable salt" is fumarate; preferably monofumarate; more preferably monofumarate hydrate; more further preferably monofumarate dihydrate.

The "HR positive (Hormone Receptor) breast cancer" herein, wherein "HR" stands for Hormone Receptor. Hormone receptors include Estrogen Receptor (ER) and Progesterone Receptor (PR).

The "HER-2 negative breast cancer" herein refers to breast cancer that is negative for Human Epidermal Growth Factor Receptor 2 (abbreviated as HER2).

The "failed standard treatment" herein refers to prior receipt of endocrine therapy with either: (a) radiological evidence of breast cancer recurrence or progression during neoadjuvant/adjuvant endocrine therapy or within 12 months after its completion; or (b) radiological evidence of progression during or after endocrine therapy for locally advanced or metastatic breast cancer.

The "locally advanced" herein refers to tumors confined to the primary organ or adjacent tissues and organs, which may have a long disease duration and large tumor size but have not metastasized to distant organs. According to the NCCN (National Comprehensive Cancer Network) Guidelines, locally advanced breast cancer refers to Stage III breast cancer. Locally advanced breast cancer is classified into resectable locally advanced breast cancer (cT3N1M0) and unresectable locally advanced breast cancer (c Any TN2M0, as well as Stage IIIB and Stage IIIC). In one embodiment, the locally advanced breast cancer is unresectable locally advanced breast cancer (c Any TN2M0, as well as Stage IIIB and Stage IIIC).

The "therapeutically effective amount" herein comprises an amount sufficient to improve or prevent the symptoms or the disease itself. In the case of cancer, a therapeutically effective dose of a drug can reduce the number of cancer cells; shrink the size of the tumor; inhibit (i.e., slow down to a certain extent and preferably stop) the infiltration of cancer cells into surrounding organs; inhibit (i.e., slow down to a certain extent and preferably stop) tumor metastasis; inhibit tumor growth to a certain extent; and/or alleviate one or more symptoms associated with the disease to a certain extent. A drug can be cytostatic and/or cytotoxic depending on the extent to which it inhibits the growth of existing cancer cells and/or kills existing cancer cells. For cancer treatment, *in vivo* efficacy can be measured by assessing overall survival duration, progression-free survival (PFS) duration, response rate (RR), duration of response, and/or quality of life.

The "MTD" herein is the Maximum Tolerated Dose.

The "DLT" herein is defined as the following toxic reactions related to the study drug (including definitely related, probably related, or possibly related) occurring in subjects of each dose group from the start of administration to the end of the first course of treatment (28 days in total): (1) Hematological toxicity: Grade 4 neutropenia lasting > 3 days; ≥ Grade 3 febrile neutropenia; Grade 4 thrombocytopenia; Grade 3 thrombocytopenia with bleeding; Grade 4 anemia; (2) Non-hematological toxicity: Grade 4 non-hematological toxicity; Grade 3 non-hematological toxicity that fails to recover to ≤ Grade 2 within 3 days after treatment (excluding asymptomatic isolated laboratory abnormalities judged by investigators to require no intervention, and excluding hyperglycemia); Grade 4 hyperglycemia with blood glucose > 27.8 mmol/L or 500 mg/dL; Grade 3 hyperglycemia with blood glucose > 13.9 mmol/L or 250 mg/dL that persists for ≥ 7 days after treatment; (3) Other toxic reactions judged by investigators to require permanent discontinuation of the study drug or resulting in the total dose of the study drug received within the DLT observation period being less than 75% of the planned dose.

The "RP2D" herein refers to Recommended Phase II Dose.

The "Objective Response Rate (ORR)" herein is defined as the proportion of subjects who achieve Complete Response (CR) or Partial Response (PR) after treatment.

The "Disease Control Rate (DCR)" herein is defined as the proportion of subjects who achieve Complete Response (CR), Partial Response (PR), and Stable Disease (SD) after treatment.

The "Duration of Response (DOR)" herein is defined as the time from the first documentation of objective response to the first occurrence of tumor progression or death from any cause.

The "Progression-Free Survival (PFS)" herein is defined as the time from the start of treatment to the first occurrence of tumor progression or death from any cause.

The "OS" (Overall Survival) herein is defined as the time from randomization to death from any cause.

The "ECOG Performance Status Score" herein refers to the Eastern Cooperative Oncology Group (ECOG) Performance Status Score (Zubrod-ECOG-WHO, ZPS, 5-point scale). It is a key indicator for assessing the general condition of cancer patients, helping predict patient prognosis, evaluating patient tolerance to various treatments, and serving as an important parameter in multiple prognostic evaluation systems.

The "AE incidence rate" herein refers to the rate of adverse events in the medical field, which are unfavorable medical occurrences that happen to patients or clinical trial subjects after receiving a drug.

The "synergistic effect" herein refers to a phenomenon where the combined effect of two co-administered drugs is more potent than their individual effects, as opposed to antagonistic effect.

The compound of Formula (I) or the compound of Formula (A) herein is a potent AKT inhibitor, whose preparation method is published in International Application WO2020156437A1. The "compound" herein comprises all stereoisomers and tautomers.

The compounds of the present disclosure can be asymmetric, for example, having one or more stereoisomers. Unless otherwise specified, all stereoisomers are included, such as enantiomers and diastereomers. The compounds of the present disclosure comprising asymmetric carbon atoms can be isolated in optically pure form or racemic form. The optically pure form can be resolved from racemic mixtures or synthesized using chiral starting materials or chiral reagents. Racemates, diastereomers, and enantiomers are all included within the scope of the present disclosure.

The "fulvestrant" herein comprises stereoisomers, solvates thereof, or pharmaceutically acceptable salts thereof.

Herein, unless otherwise specified, when referring to the dosage of the compound of Formula (A) or fulvestrant, it refers to the dosage of the active compound (free base). It is understood that when administered as a pharmaceutically acceptable salt or a hydrate, the dosage or the ratio between the two can still be calculated as the active compound.

## DETAILED EMBODIMENTS

The following typical examples are used to illustrate the present disclosure, so as to show the advantageous activity or beneficial technical effects of the composition of the present disclosure. However, it should be understood that the following test protocols are merely examples of the content of the present disclosure, rather than limiting the scope of the present disclosure. Simple substitutions or improvements made by those skilled in the art to the present disclosure shall all fall within the scope of the technical solutions protected by the present disclosure.

### **Example 1: Experiment on the inhibition of breast cancer cell proliferation by the compound of Formula** (A) **alone or in combination with fulvestrant**

1. Experimental Objective:

To evaluate the effect of the compound of Formula (A) alone or in combination with fulvestrant on the proliferation of 5 cell lines.

2. Experimental Methods

2.1 Cell Culture

a). BT-474 cells were cultured in Hybri-Care medium supplemented with 1% antibiotics and 10% FBS, and incubated in the condition of 37°C with 5% $CO_2$; b). MCF-7 cells were cultured in EMEM medium supplemented with 1% antibiotics, 0.01 mg/mL insulin, and 10% FBS, and incubated in the condition of 37°C with 5% $CO_2$; c). ZR-75-1 cells were cultured in RPMI-1640 medium supplemented with 1% antibiotics and 10% FBS, and incubated in the condition of 37°C with 5% $CO_2$; d). T47D cells were cultured in RPMI-1640 medium supplemented with 1% antibiotics, 0.2 U/mL insulin, and 10% FBS, and incubated in the condition of 37°C with 5% $CO_2$; e) CAMA-1 cells were cultured in EMEM medium supplemented with 1% antibiotics and 10% FBS, and incubated in the condition of 37°C with 5% $CO_2$.

2.2 Cell Plating

a). Cells were routinely cultured until reaching 80%-90% confluence and when the required cell number was reached, they were harvested; b). Resuspended in the corresponding medium, counted, and prepared into a cell suspension with appropriate density; c). The cell suspension was added to a 384-well plate at 40 μL per well.

Table 1. Plating densities of different cell lines

| Number | Cell | Density (per well) |
|---|---|---|
| 1 | BT-474 | 1000 |
| 2 | MCF-7 | *500* |
| 3 | ZR-75-1 | 500 |
| 4 | T47D | 500 |
| 5 | CAMA-1 | 1000 |

d). The cells were cultured overnight in an incubator at 37°C with 5% $CO_2$ .

2.3 Preparation of Compounds

a). The compound of Formula (A) was serially diluted 3-fold for 10 concentrations starting from a stock solution of 60 mM (using the monofumarate dihydrate of the compound of Formula (A), with the concentration calculated based on the free base of the compound of Formula (A); the compound of Formula (A) or salt thereof was prepared with reference to the preparation method disclosed in WO2022017448A1); b). Fulvestrant was diluted from 10 mM to 2 mM with DMSO, then serially diluted 3-fold for 10 concentrations; c). Fulvestrant was diluted from 2 mM to 0.2 mM with DMSO, then serially diluted 3-fold for 10 concentrations; d). Fulvestrant was diluted from 0.2 mM to 2 μM with DMSO; e). Fulvestrant was diluted from 2 μM to 0.8 μM with DMSO; f). Fulvestrant was diluted from 2 μM to 0.3 μM with DMSO; g) Fulvestrant was diluted from 0.8 μM to 0.08 μM with DMSO; h). The final concentration of DMSO was 0.1%; i). Cells supplemented with DMSO served as control wells for high reading values; j). Cell-free medium served as control wells for low reading values.

2.4 Compound Treatment of Cells

a). Monotherapy:

24 Hours after BT474 and ZR-75-1 cells were plated, the compound was administered alone. For the compound of Formula (A): 20 nL of the compound prepared in step 2.3 a) + 20 nL of DMSO were added. For the compound fulvestrant: 20 nL of the compound prepared in step 2.3 b) + 20 nL of DMSO were added.

24 Hours after MCF-7, T47D, and CAMA-1 cells were plated, the compound was administered alone. For the compound of Formula (A): 20 nL of the compound prepared in step 2.3 a) + 20 nL of DMSO were added. For compound fulvestrant: 20 nL of the compound prepared in step 2.3 c) + 20 nL of DMSO were added.

b) Combination Therapy:

24 Hours after BT474 and ZR-75-1 cells were plated, the compound of Formula (A) was administered in combination with fulvestrant: 20 nL of the compound prepared in step 2.3 a) + 20 nL of the compounds prepared in step 2.3 b) and d).

24 Hours after MCF-7 cells were plated, the compound of Formula (A) was administered in combination with fulvestrant: 20 nL of the compound prepared in step 2.3 a) + 20 nL of the compounds prepared in step 2.3 c), e), and f).

24 Hours after T47D cells were plated, the compound of Formula (A) was administered in combination with fulvestrant: 20 nL of the compound prepared in step 2.3 a) + 20 nL of the compounds prepared in step 2.3 c), e), and g).

24 Hours after CAMA-1 cells were plated, the compound of Formula (A) was administered in combination with fulvestrant: 20 nL of the compound prepared in step 2.3 a) + 20 nL of the compound prepared in step 2.3 c).

c) Detection of the Final Concentrations of Compounds:

1) BT474 and ZR-75-1 cells:

Gradient concentrations of the compound of Formula (A) (nM): 30000, 10000, 3333, 1111, 370, 123, 41.2, 13.7, 4.57, 1.52 nM.

Fulvestrant for point-to-point combination (nM): 1000, 333, 111, 37, 12.3, 4.1, 1.37, 0.46, 0.15, 0.05, which are respectively combined with the gradient concentrations of the compound of Formula (A);

Fulvestrant for single-concentration combination (nM): 1000, 1, which are respectively combined with the gradient concentrations of the compound of Formula (A).

2) MCF-7 cells:

The compound of Formula (A) (nM): 30000, 10000, 3333, 1111, 370, 123, 41.2, 13.7, 4.57, 1.52;

Fulvestrant for point-to-point combination (nM): 100, 33.3, 11.1, 3.7, 1.23, 0.41, 0.14, 0.046, 0.015, 0.005, which are respectively combined with the gradient concentrations of the compound of Formula (A);

Fulvestrant for single-concentration combination (nM): 0.4, 0.15, which are respectively combined with the gradient concentrations of the compound of Formula (A).

3) T47D cells:

The compound of Formula (A) (nM): 30000, 10000, 3333, 1111, 370, 123, 41.2, 13.7, 4.57, 1.52;

Fulvestrant for point-to-point combination (nM): 100, 33.3, 11.1, 3.7, 1.23, 0.41, 0.14, 0.046, 0.015, 0.005, which are respectively combined with the gradient concentrations of the compound of Formula (A);

Fulvestrant for single-concentration combination (nM): 0.4, 0.04, which are respectively combined with the gradient concentrations of the compound of Formula (A).

4) CAMA-1 cells:

The compound of Formula (A) (nM): 30000, 10000, 3333, 1111, 370, 123, 41.2, 13.7, 4.57, 1.52;

Fulvestrant for point-to-point combination (nM): 100, 33.3, 11.1, 3.7, 1.23, 0.41, 0.14, 0.046, 0.015, 0.005, which are respectively combined with the gradient concentrations of the compound of Formula (A);

Fulvestrant for single-concentration combination (nM): 1.2, 0.14, which are respectively combined with the gradient concentrations of the compound of Formula (A).

d). The cell culture plates were centrifuged at 1000 rpm for 1 min, then placed in an incubator at 37°C with 5% $CO_2$ for 7 days.

2.5 Detection by CTG Method

a). 40 μL of CTG reagent (CelltiterGlo kit) was added to each well, placed on a rapid shaker for 2 minutes of oscillation, centrifuged at 1000 rpm for 1 min, and then left at room temperature in the dark for 30 minutes; b). The chemiluminescent signal values were read using an Envision instrument.

2.6 Data Analysis

Using the inhibition rate data measured by monotherapy or single-concentration combination therapy in Section 2.4, $IC_{50}$ was calculated with GraphPad Prism 8 software. The $IC_{50}$ (half-maximal inhibitory concentration) of the compound was obtained using the following nonlinear fitting formula: Y=Minimum+(Maximum - Minimum)/(1+10^((Log$IC_{50}$-X)*Slope)).

X: Logarithm of compound concentration; Y: Inhibition rate (%inhibition).

Inhibition rate (%inhibition)=100-(Reading of compound well-Reading of low control well)/(Reading of high control well-Reading of low control well)] * 100

2.7 $IC_{50}$ for Cell Proliferation Activity

The determination results of $IC_{50}$ for cell proliferation activity were shown in Tables 2-5 below.

Table 2. Results of the study on the inhibitory effect of the compound of Formula (A) in combination with fulvestrant on the proliferation of BT-474 and ZR-75-1 cells

| **Compound** | **BT-474 Cells/$IC_{50}$ (nM)** | **Maximum Inhibition Rate on BT-474 Cells (%)*** | **ZR-75-1 Cells/$IC_{50}$ (nM)** | **Maximum Inhibition Rate on ZR-75-1 Cells (%)*** |
|---|---|---|---|---|
| The compound of Formula (A) | 668.38 | 84.62 | 2169.53 | 92.45 |
| Fulvestrant | > 1000 | 43.33 | >1000 | 43.96 |
| The compound of Formula (A) +1 μM Fulvestrant | 10.60 | 88.33 | < 1.52 | 95.60 |

(continued)

| Compound | BT-474 Cells/$IC_{50}$ (nM) | Maximum Inhibition Rate on BT-474 Cells (%)* | ZR-75-1 Cells/$IC_{50}$ (nM) | Maximum Inhibition Rate on ZR-75-1 Cells (%)* |
|---|---|---|---|---|
| The compound of Formula (A) +1 nM Fulvestrant | 153.20 | 86.45 | 33.90 | 94.13 |
| Note: *The maximum inhibition rate is the maximum value automatically fitted when calculating $IC_{50}$ using GraphPad Prism 8 software. | | | | |

Table 3. Results of the study on the inhibitory effect of the compound of Formula (A) in combination with fulvestrant on MCF-7 cells proliferation

| Compound | MCF-7 Cells/$IC_{50}$ (nM) | Maximum Inhibition Rate on MCF-7 Cells (%)* |
|---|---|---|
| The compound of Formula (A) | 758.57 | 98.92 |
| Fulvestrant | 0.19 | 54.67 |
| The compound of Formula (A) +0.4 nM Fulvestrant | ≤1.5 | 98.13 |
| The compound of Formula (A) +0.15 nM Fulvestrant | 6.91 | 98.77 |
| Note: *The maximum inhibition rate is the maximum value automatically fitted when calculating $IC_{50}$ using GraphPad Prism 8 software. | | |

Table 4. Results of the study on the inhibitory effect of compound of Formula (A) in combination with fulvestrant on T47D cells proliferation

| Compound | T47D Cells/$IC_{50}$ (nM) | Maximum Inhibition Rate on T47D Cells (%)* |
|---|---|---|
| The compound of Formula (A) | 358.23 | 91.47 |
| Fulvestrant | 0.09 | 62.71 |
| The compound of Formula (A) +0.4 nM Fulvestrant | <1.52 | 94.43 |
| The compound of Formula (A) +0.04 nM Fulvestrant | 64.03 | 92.19 |
| Note: *The maximum inhibition rate is the maximum value automatically fitted when calculating $IC_{50}$ using GraphPad Prism 8 software. | | |

Table 5. Results of the study on the inhibitory effect of the compound of Formula (A) in combination with fulvestrant on CAMA-1 cells proliferation

| Compound | CAMA-1 Cells/$IC_{50}$ (nM) | Maximum Inhibition Rate on CAMA-1 Cells (%)* |
|---|---|---|
| The compound of Formula (A) | 1131.45 | 95.43 |
| Fulvestrant | >100 | / |
| The compound of Formula (A) +1.2 nM Fulvestrant | 80.65 | 98.74 |
| The compound of Formula (A) +0.14nM Fulvestrant | 129.13 | 98.42 |
| Note: *The maximum inhibition rate is the maximum value automatically fitted when calculating $IC_{50}$ using GraphPad Prism 8 software. | | |

Conclusion: The compound of Formula (A) alone and in combination with fulvestrant respectively exhibited excellent *in vitro* antiproliferative activity against five cell lines (BT-474, MCF-7, ZR-75-1, T47D, CAMA-1).

2.8 Compounds Combination Evaluation Index (Synergy.score)

Detection Method: The inhibition rate data measured by point-to-point combination in Section 2.4 were imported into the specific calculation template of the SynergyFinder website (https://tangsoftwarelab.shinyapps.io/synergyfinder/#!/dashboard.), uploaded, and analyzed for the combination index.

Evaluation Criteria: Synergy.score < -10, the interaction between the two compounds is antagonistic;

-10 < Synergy.score < 10, the interaction between the two compounds is additive;

Synergy.score > 10, the interaction between the two compounds is synergistic.

The specific compounds combination evaluation indices were shown in the following Table:

Table 6. Compounds combination indices of different cell lines

| Cell Line | Drug Combination | Synergy.score |
|---|---|---|
| BT-474 | The compound of Formula (A) +Fulvestrant | 7.055 |
| MCF-7 | The compound of Formula (A) +Fulvestrant | 10.249 |
| ZR-75-1 | The compound of Formula (A) +Fulvestrant | 10.412 |
| T47D | The compound of Formula (A) +Fulvestrant | 11.299 |
| CAMA-1 | The compound of Formula (A) +Fulvestrant | 16.837 |

Conclusion: The combined use of the compound of Formula (A) and fulvestrant exhibited a synergistic effect on inhibiting the proliferation of breast cancer cells such as MCF-7, ZR-75-1, T47D, and CAMA-1.

**Example 2: Study on the Tumor Inhibition Rate of the Compound of Formula (A) in Combination with Fulvestrant in the MCF-7 Human Breast Cancer Subcutaneous Xenograft Model**

1. Experimental Objective:

The purpose of this study is to evaluate the therapeutic efficacy of the compound of Formula (A) in combination with fulvestrant in the MCF-7 human breast cancer subcutaneous xenograft model in female BALB/c nude mice.

2. Experimental Materials and Equipment

2.1 Experimental Animals:

BALB/c nude mice, 6-8 weeks old, female, weighing 18-20 g, were purchased from Beijing Anikeeper Biotech Co., Ltd.. The feeding environment was SPF-grade, the temperature is (23±3)°C and the humidity is 40-70%.

2.2 Test Articles

2.2.1 Solvent Selection:

Solvent for the compound of Formula (A): 1,2-Propanediol (PG) : 50% Solutol HS-15 : 0.5% Hydroxypropyl Methylcellulose (HPMC) = 2 : 1 : 7.

2.2.3 Preparation Methods were shown in the following Table:

Table 7. Compound preparation methods

| Drug | Preparation Method | Concentration (mg/mL) | Storage |
|---|---|---|---|
| Solvent | 50% Solutol HS-15: An appropriate amount of Solutol HS-15 solid was weighed and heated to dissolve. Then | - | Stored at room temperature |

(continued)

| Drug | Preparation Method | Concentration (mg/mL) | Storage |
|---|---|---|---|
| | 250 g of Solutol HS-15 liquid was accurately weighed, ultrapure water was added to 500 mL, and the mixture was vortexed to mix well. A 50% Solutol HS-15 aqueous solution was obtained and stored at room temperature for later use.<br>0.5% HPMC (Hydroxypropyl Methylcellulose): 2.5 g of HPMC was weighed, ultrapure water was added to 500 mL, and the mixture was vortexed to mix well. A 0.5% HPMC aqueous solution was obtained and stored at room temperature for later use.<br>1,2-PG & 50% Solutol HS-15 & 0.5% HPMC: Appropriate volumes of propylene glycol (1,2-PG), 50% Solutol HS-15 aqueous solution, and 0.5% HPMC aqueous solution were pipetted respectively and mixed uniformly. The ratio of 1,2-PG, 50% Solutol HS-15 aqueous solution, and 0.5% HPMC aqueous solution in the mixture was 20:10:70 (v:v:v). It was used as a blank solvent and stored at room temperature. | | |
| The compound of Formula (A) | 20.1 mg of the compound of Formula (A) powder was weighed (the monofumarate dihydrate of the compound of Formula (A) was used; the compound of Formula (A) or salt thereof was prepared with reference to the preparation method disclosed in WO2022017448A1, with a conversion factor of 74.28%). Then 1.194 mL of PG was added, and the mixture was vortexed and sonicated to mix well. Subsequently, 0.597 mL of 50% Solutol HS-15 aqueous solution was added, and the mixture was vortexed and sonicated to mix well. Finally, 4.180 mL of 0.5% | 2.5 | Prepared and used immediately |
| | HPMC solution was added, and the mixture was vortexed and sonicated to mix well to form a homogeneous solution. | | |
| Fulvestrant | 2.8 mL of 50 mg/mL Fulvestrant injection was pipetted and used directly. | 50 | Prepared and used immediately |

3. Experimental Methods and Procedures

3.1 Cell Culture

MCF-7 tumor cells were cultured in DMEM medium containing inactivated 15% fetal bovine serum, 100 U/mL penicillin, and 100 μg/mL streptomycin in an incubator at 37°C with 5% $CO_2$. Tumor cells in the logarithmic growth phase were used for *in vivo* tumor inoculation.

3.2 Inoculation and Grouping of Tumor Cells

One day before the inoculation of MCF-7 tumor cells, the mice were subcutaneously implanted with an estrogen tablet (0.5 mg, 90-day release period) on the left side. MCF-7 tumor cells resuspended in serum-free DMEM medium were inoculated subcutaneously into the right costal region of the experimental animals at a density of $1.5 \times 10^7$ cells (0.2 mL + Matrigel), with a total of 72 animals inoculated. When the tumors grew to approximately 204 $mm^3$, 48 animals with relatively uniform tumor volumes were selected for grouping and administration, divided into 4 groups, and administered according to the dosing schedule.

Table 8. Compounds administration schedule

| Group | Number of Cases | Treatment | Dose (mg/kg) | Administration Volume (μL/g) | Drug Concentration (mg/mL) | Administration Route | Treatment Cycle |
|---|---|---|---|---|---|---|---|
| 1 | 12 | Blank Control | - | 10 | - | p.o. | QD x 4 weeks |
| 2 | 12 | The compound of Formula (A) | 25 | 10 | 2.5 | p.o. | QD x 4 weeks |
| 3 | 12 | Fulvestrant | 5 mg/mouse | 100 μL/mouse | 50 | s.c. | QW x 4 weeks |
| 4 | 12 | The compound of Formula (A) | 25 | 10 | 2.5 | p.o. | QD x 4 weeks |
| | | Fulvestrant | 5 mg/mouse | 100 μL/mouse | 50 | s.c. | QW x 4 weeks |
| Note: p.o. means oral administration; s.c. means subcutaneous injection; QD x 4 weeks means once daily administration for four consecutive weeks; QW x 4 weeks means once weekly administration for four consecutive weeks. | | | | | | | |

3.3 Detection Indicators

3.3.1 Tumor Volume:

Tumor volume was measured twice a week using a vernier caliper. The long diameter and short diameter of the tumor were measured, and the volume was calculated using the formula: Volume = 0.5×long diameter×short diameter$^2$.

3.3.2 Animal Response After Administration:

While measuring the tumor volume, the body weight of the mice was weighed. The relationship between the changes in the mice's body weight and the administration time was recorded. Meanwhile, the survival status and health conditions of the mice were observed, such as their general states including activity and food intake during the administration period.

3.4 Drug Evaluation Indicators

3.4.1 Relative Tumor Volume Increase Ratio T/C (%)

T/C (%) = Mean relative tumor volume of treatment group/Mean relative tumor volume of control group × 100

3.4.2 Tumor Growth Inhibition Rate (TGI, %)

$$\text{Tumor Growth Inhibition Rate (TGI, \%)} = (1 - \text{T/C}) \times 100$$

3.5 Statistical Analysis

3.5.1 Data Collection:

Measurements and observations were performed as required by the experimental protocol, with records made

manually or directly entered into a computer database.

3.5.2 Statistical Analysis:

Statistical analysis of tumor volume between groups was performed using the statistical software SPSS 16.0 and One-Way ANOVA test. A p-value < 0.05 was considered statistically significant.

4. Experimental Results

Table 9. Pharmacodynamic data of the compound of Formula (A) in combination with fulvestrant in the MCF-7 human breast cancer subcutaneous xenograft model

| Group | Number of Animals (n) | Body weight (g)[a] | | Tumor Volume at Grouping ($mm^3$)[a] | Tumor Volume 28 Days After Administration ($mm^3$) | T/C (%) | TGI (%) | P value[b,c] |
|---|---|---|---|---|---|---|---|---|
| | | Pre-administration | Post 28 Days of Treatment | | | | | |
| G1: Vehicle Control | 12 | 24.4 ± 0.4 | 24.8 ± 0.8 | 204 ± 6 | 1,160 ± 76 | - | - | - |
| G2: The compound of Formula (A) 25 mg/kg | 12 | 24.6 ± 0.3 | 23.8 ± 0.5 | 204 ± 6 | 684 ± 65 | 58.4 | 41.6 | <0.001 |
| G3: Fulvestrant 5mg/mouse | 12 | 24.7 ± 0.2 | 25.9 ± 0.3 | 204 ± 6 | 546 ± 54 | 46.9 | 53.1 | <0.001 |
| G4: The compound of Formula (A) (25mg/kg) +Fulv estrant (5 mg/mouse) | 12 | 24.7 ± 0.3 | 23.5 ± 0.4 | 204 ± 6 | 455 ± 45 | 39.0 | 61.0 | <0.001 |
| Note: a means Mean ± standard error; b means compared with the control group; c means when One-way ANOVA combined with LSD test was used, and a P value < 0.05 was considered statistically significant. | | | | | | | | |

5. Experimental Conclusion: The 25 mg/kg compound of Formula (A) monotherapy group, 5 mg/mouse fulvestrant monotherapy group, and their combination therapy group all exhibited a significant inhibitory effect on tumor growth. Compared with each monotherapy group, the combination therapy group showed a better enhanced effect in inhibiting tumor growth.

**Example 3: Pharmacodynamic Study of the compound of Formula** (A) **in Combination with Fulvestrant in the MCF-7 Human Breast Cancer Subcutaneous Xenograft Model**

1. Experimental Objective

The purpose of this study was to evaluate the therapeutic efficacy of the compound of Formula (A) in combination with fulvestrant in the MCF-7 human breast cancer subcutaneous xenograft model in female BALB/c nude mice.

2. Experimental Protocol

On the 28th day of treatment for the mice in Experiment 2, animal plasma was continuously collected for PK detection, with a total of 112 plasma samples and 16 tumor samples. The detailed sampling protocol is shown in the following Table:

Table 10. PK sample sampling protocol

| Group | Number of Cases | Treatment | Dose (mg/kg) | Number of Samples | Sample Collection |
|---|---|---|---|---|---|
| 1 | 8 | Control | - | 4 | Plasma and tumors were collected at 4h |
| 2 | 8 | The compound of Formula (A) | 25 | 4 | Plasma was collected at 0h, 0.5h, 1h, 2h, 4h, 8h, 24h , 48h, 72h |
| | | | | 4 | Tumors was collected at 4h |
| 3 | 8 | Fulvestrant | 5 mg/mouse | 4 | Plasma was collected at 0h, 0.5h, 1h, 2h, 4h, 8h, 24h , 48h, 72h |
| | | | | 4 | Tumors was collected at 4h |
| 4 | 8 | The compound of Formula (A) | 25 | 4 | Plasma was collected at 0h, 0.5h, 1h, 2h, 4h, 8h, 24h , 48h, 72h |
| | | Fulvestrant | 5 mg/mouse | 4 | Tumors was collected at 4h |
| Number of Plasma Samples (for PK Detection) | | | | 112 | |
| Number of Tumor Samples | | | | 16 | |

3. Experimental Results

Table 11. Pharmacokinetic parameters of the compound of Formula (a) in plasma

| PK parameters | Unit | G2: The compound of Formula (A) 25 mg/kg | G4: The compound of Formula (A) 25 mg/kg + Fulvestrant 5 mg/mouse |
|---|---|---|---|
| $T_{1/2}$ | h | 7.93 | 8.3 |
| $T_{max}$ | h | 2.75 | 5.00 |
| $C_{max}$ | ng/mL | 1336 | 1120 |
| $AUC_{last}$ | h*ng/mL | 18525 | 20319 |
| $AUC_{Inf}$ | h*ng/mL | 18778 | 20736 |
| $AUC_{\%Extrap_}obs$ | % | 1.48 | 2.37 |
| $MRT_{Inf_}obs$ | h | 10.5 | 12.6 |
| $AUC_{last}/D$ | h*mg/mL | 741 | 813 |

Table 12. Pharmacokinetic parameters of fulvestrant in plasma

| PK parameters | Unit | G3: Fulvestrant 5 mg/mouse | G4: The compound of Formula (A) 25 mg/kg + Fulvestrant 5 mg/mouse |
|---|---|---|---|
| $T_{1/2}$ | h | 23.6 | 24.0 |
| $T_{max}$ | h | 0.375 | 0.250 |
| $C_{max}$ | ng/mL | 268 | 231 |
| $AUC_{last}$ | h*ng/mL | 5784 | 4831 |
| $AUC_{Inf}$ | h*ng/mL | 6610 | 5626 |
| $AUC_{\%Extrap_}obs$ | % | 12.6 | 14.1 |
| $MRT_{Inf_}obs$ | h | 37.7 | 42.0 |
| $AUC_{last}/D$ | h*mg/mL | 145 | 121 |

The 25 mg/kg compound of Formula (A) monotherapy group, 5 mg/mouse fulvestrant monotherapy group, and their combination therapy group all exhibited significant tumor growth inhibitory effects. Compared with each monotherapy group, the combination therapy group showed a certain enhancement in tumor growth inhibition. Concomitant PK study results indicated that after the combination treatment of the compound of Formula (A) with fulvestrant in the MCF-7 human breast cancer subcutaneous xenograft model, neither the compound of Formula (A) nor fulvestrant had a significant impact on their respective plasma pharmacokinetic parameters, suggesting that no obvious plasma pharmacokinetic interaction occurred between the compound of Formula (A) and fulvestrant in this model.

**Example 4: Evaluation of Safety, Tolerability, Pharmacokinetic Characteristics and Preliminary Efficacy of the Compound of Formula** (A) **Tablets in Patients with Advanced Malignant Solid Tumors**

All contents of the trial protocol with registration number CTR20211999 on the China Drug Clinical Trial Registration and Information Disclosure Platform and results thereof are incorporated herein by reference.

Study Objectives: To evaluate the safety and tolerability of the compound of Formula (A) in patients with advanced malignant solid tumors (including breast cancer and ovarian cancer) who have failed standard treatment, and to explore the maximum tolerated dose (MTD) of the compound of Formula (A), and determine the recommended phase II dose (RP2D). To evaluate the pharmacokinetic (PK) characteristics of the compound of Formula (A) following a single dose and continuous administration for 21 days in patients with advanced solid tumors. To assess the preliminary efficacy of the compound of Formula (A) in the treatment of advanced solid tumors.

Trial Design: The study was an open-label, single-arm, non-randomized clinical trial focusing on safety, efficacy, and pharmacokinetics. The first part was the dose-finding phase, and the second part was the dose-expansion phase.

The first Part: Dose-Finding Phase

A starting dose of 20 mg of the compound of Formula (A) was selected, with successive dose-finding groups set as 20 mg, 50 mg, 100 mg, 200 mg, 300 mg, and 400 mg. The administration regimen included once-daily single-dose administration (with a 7-day washout period) and multiple-dose administration (continuous administration for 21 days, 28 days as one cycle, treatment continued until intolerable toxicity, disease progression, or subject withdrawal).

The second Part: Dose-Expansion Phase

The dose-expansion phase was initiated after the completion of the dose-finding phase. Based on the results of dose escalation, the dosage for the dose-expansion group was determined, with oral administration once daily (continuous administration for 21 days, 28 days as one cycle, and treatment will be continued until intolerable toxicity, disease progression, or subject withdrawal).

Endpoint Indicators

1) Safety and Tolerability: Maximum Tolerated Dose (MTD) and Dose-Limiting Toxicity (DLT);
2) PK Indicators: See Tables 13 and 14 below;
3) Efficacy Indicators: Objective Response Rate (ORR), Disease Control Rate (DCR), Duration of Response (DOR), and Progression-Free Survival (PFS).

Table 13. PK Indicators of the compound of Formula (A) monotherapy in the dose-finding phase

| **Single Dose** | **400mg Monotherapy ’(n=10)** |
|---|---|
| $t_{1/2}$ (h) | 7.216±1.91 |
| $T_{max}$ (h) | 3 [1.00,4.00] |
| $C_{max}$ (ng/ml) | 392.3±178.4 |
| $AUC_{0-t}$ (h*ng/ml) | 2377.372±1155.555 |
| Vz/F (L) | 1919.972±1004.745 |
| CL/F (L/h) | 186.378±99.471 |

Table 14. PK Indicators of the compound of Formula (A) monotherapy in the dose-expansion phase

| Multiple Doses | 400mg Monotherapy (n=8) |
|---|---|
| $t_{1/2}$ (h) | 12.95±4.19 |
| $T_{max,ss}$ (h) | 3.00 [0,6.00] |
| $C_{max,ss}$ (ng/mL) | 462.24±244.69 |
| $C_{min,ss}$ (ng/mL) | 33.76±14.66 |
| $C_{av,ss}$ (ng/mL) | 131.73±64.21 |
| $AUC_{0-\tau}$ (h·ng/mL) | 3161.62±1540.95 |
| $AUC_{0-t,ss}$ (h·ng/mL) | 3534.6±1774.59 |
| $CL_{ss}/F$ (L/h) | 155.88±81.87 |
| $V_{ss}/F$ (L) | 2884.97±1671.38 |

**Example 5: Safety, Tolerability, Pharmacokinetic Properties and Preliminary Efficacy Evaluation of the compound of Formula (A) Tablets in Combination with Fulvestrant in Patients with Locally Advanced or Metastatic HR Positive and HER-2 Negative Breast Cancer with Failed Standard Treatment**

Study Objective: To evaluate the safety and tolerability of the compound of Formula (A) in combination with fulvestrant in patients with locally advanced or metastatic HR positive/HER-2 negative breast cancer, and to determine the recommended dose for subsequent clinical trials as well as the preliminary efficacy of the combination therapy.

Study Design: The study was divided into two phases: the first phase was the dose-finding phase, and the second phase was the dose-expansion phase.

A starting dose of 400 mg was selected for the compound of Formula (A) in combination with fulvestrant in dose-finding phase. Based on the safety and tolerability results of the 400 mg dose group, dose escalation to the 500 mg dose group was conducted. The administration regimen for the compound of Formula (A) was once daily; continuous administration was performed for 21 days, followed by a 7-day rest period, with 28 days as one administration cycle. The combination drug fulvestrant was administered according to standard treatment. It was given by intramuscular injection, once on Day 1 of each cycle, at a dose of 500 mg (administered *via* two separate intramuscular injections, 250 mg per buttock). An additional 500 mg dose was administered two weeks after the initial dose, with 28 days as one administration cycle. During the dose-finding phase, dose-limiting toxicity (DLT) was observed in subjects, and the DLT observation period was from the start of administration to the end of the first cycle.

The second Part is dose-expansion phase. After the preliminary confirmation of the safety of the 400 mg and 500 mg dose levels in the dose-finding phase, the dose-expansion phase was initiated to further evaluate the safety, tolerability, and preliminary anti-tumor efficacy of the compound of Formula (A) in combination with fulvestrant. The administration regimen for subjects was the same as that in the dose-finding phase: 400 mg or 500 mg of the compound of Formula (A) was orally administered once daily, with continuous administration for 21 days followed by a 7-day rest period, and 28 days as one administration cycle. Fulvestrant, the combined drug, was administered by intramuscular injection once on Day 1 of each cycle at a dose of 500 mg (administered *via* two separate intramuscular injections, 250 mg per buttock). An additional 500 mg dose was administered two weeks after the initial dose, with 28 days as one administration cycle. All subjects could continue administration until disease progression, intolerable toxicity, voluntary withdrawal of subjects, subject death, or loss to follow-up (whichever occurred first).

Study Results: In the dose-finding phase, all 6 subjects receiving the combination therapy completed the dose-limiting toxicity (DLT) observation, and no DLT events occurred.

(1) Safety Data of Combination Therapy: Safety analysis was performed on subjects who had received the drugs, showing that the overall safety of the combination administration was good and controllable, with types of adverse events similar to those of drugs targeting the same pathway. The incidence of treatment-related adverse events (TRAE) was 100%, most of which were grade 1-2 adverse events. No grade ≥3 adverse events or serious adverse events were reported. Common TRAEs mainly included diarrhea (100%), vomiting (75.0%), hypercholesterolemia (62.5%), hyperglycemia (50.0%), nausea (50.0%), increased glycated hemoglobin (37.5%), increased AST/ALT (37.5%), proteinuria (37.5%), increased lipase (37.5%), hypoalbuminemia (25.0%) and etc.

(2) Preliminary Efficacy of Combination Therapy: Tumor assessment was performed on 5 subjects in the dose-expansion phase after treatment. Among them, 1 subject had no measurable target lesions, with an overall efficacy assessment of stable disease (SD); 1 subject achieved confirmed partial response (PR), with the tumor lesion reduced from 32.9 mm at baseline to 16 mm; 3 subjects had SD, among which 1 subject's tumor lesion reduced from 100 mm at baseline to 83 mm.

The disease control rate (DCR) was 100% (5/5), and the objective response rate (ORR) was 20% (1/5). Currently, all enrolled subjects are still receiving treatment in the study, with no cases were withdrawn.

(3) Pharmacokinetic (PK) Data: During the dose-finding and dose-expansion phases, blood samples were collected after administration to detect the corresponding indicators. Currently, PK data statistics for a total of 6 subjects in the 400 mg dose group have been completed. Comparison of the available data showed the pharmacokinetic data of the compound of Formula (A) as monotherapy and in combination with fulvestrant in the 400 mg dose group. Detailed PK data summary was shown in Tables 15 and 16 below.

Table 15. PK Indicators of the compound of Formula (A) in combination with fulvestrant during the dose-finding phase

| Single Dose | 400mg in Combination (n=6) |
|---|---|
| $t_{1/2}$ (h) | 6.141±1.423 |
| $T_{max}$ (h) | 1.50[0.5, 2.0] |
| $C_{max}$ (ng/ml) | 447.167±335.515 |
| $AUC_{0-t}$ (h*ng/ml) | 1959.285±1298.071 |
| Vz/F (L) | 2035.032±890.409 |
| CL/F (L/h) | 237.932±100.327 |

Table 16. PK Indicators of the compound of Formula (A) in combination with fulvestrant during the dose-expansion phase

| Multiple Doses | 400mg in Combination (n=6) |
|---|---|
| $t_{1/2}$ (h) | 7.353±0.4 |
| $T_{max,ss}$ (h) | 2.5[0.5, 3] |
| $C_{max,ss}$ (ng/mL) | 513.667±265.483 |
| $C_{min,ss}$ (ng/mL) | 33.650±21.623 |
| $C_{av,ss}$ (ng/mL) | 134.188±75.036 |
| $AUC_{0_\tau}$ (h·ng/mL) | 3220.504±1800.872 |
| $AUC_{0-t,ss}$ (h·ng/mL) | 3220.504±1800.872 |
| $CL_{ss}$/F (L/h) | 154.668±73.112 |
| $V_{ss}$/F (L) | 1625.806±746.244 |

**Example 6: Study on the Efficacy and Safety of the Compound of Formula (A) Tablets in Combination with Fulvestrant in Patients with Locally Advanced or Metastatic HR Positive and HER-2 Negative Breast Cancer with Failed Standard Treatment**

Study Objective: To evaluate the anti-tumor efficacy and safety of the compound of Formula (A) in combination with fulvestrant versus placebo in combination with fulvestrant in the overall population of patients with locally advanced or metastatic HR positive and HER-2 negative breast cancer, as well as in the subgroup population with PIK3CA/AKT1/PTEN alterations.

Study Design: This study was designed as a randomized, controlled, double-blind, multicenter trial. The enrolled population consisted of patients with recurrent locally advanced or metastatic HR positive and HER-2 negative breast cancer who had previously received first-line to second-line systemic therapy (with at least one line being endocrine therapy). Eligible subjects were randomly assigned to either the compound of Formula (A) in combination of fulvestrant group or the placebo in combination of fulvestrant group at a 1:1 ratio. The proposed administration regimen for the compound of Formula (A) was 400 mg or 500 mg once daily, with continuous administration for 21 days followed by a 7-day rest period, and 28 days as one administration cycle. The actual administration dose and frequency might be adjusted based on the results of exploratory studies and quantitative pharmacometric model analysis.

The combined drug fulvestrant was administered in accordance with standard treatment *via* intramuscular injection. It was given once on Day 1 of each cycle at a dose of 500 mg (administered as two separate intramuscular injections, 250 mg per buttock). An additional 500 mg dose was administered two weeks after the initial dose, with a 28-day administration cycle.

Endpoint Indicators: The primary endpoint was the progression-free survival (PFS) in the overall population and the subgroup population with PIK3CA/AKT1/PTEN alterations, as assessed by investigators in accordance with the RECIST 1.1 criteria. The key secondary endpoint was the overall survival (OS) in the overall population and the subgroup population with PIK3CA/AKT1/PTEN alterations. All subjects could continue administration until disease progression, intolerable toxicity, voluntary withdrawal of subjects, subject death, or loss to follow-up (whichever occurred first).

## Claims

1. Use of a compound represented by Formula (I), or a pharmaceutically acceptable salt thereof, or a hydrate thereof, in the manufacture of a medicament for preventing or treating breast cancer, wherein the breast cancer is hormone receptor positive breast cancer,

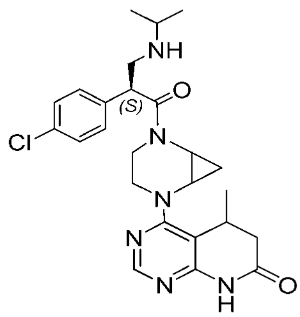

Formula (I) .

2. The use according to claim 1, wherein the compound of Formula (I) is the compound of Formula (A) or the compound of Formula (B):

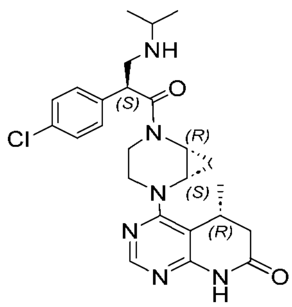

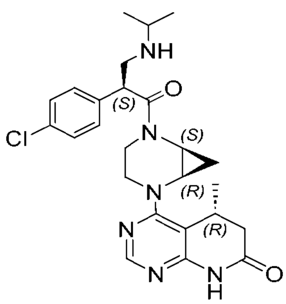

Formula (A) Formula (B) .

3. The use according to claim 1 or 2, wherein the compound of Formula (A) or pharmaceutically acceptable salt thereof or hydrate thereof is used in combination with fulvestrant.

4. The use according to any one of claims 1-3, wherein the breast cancer is HR positive breast cancer; preferably is HR positive and HER-2 negative breast cancer; more preferably is ER positive, PR positive and HER-2 negative hormone receptor positive breast cancer; further preferably is HR positive and HER-2 negative breast cancer which is refractory to standard treatment, locally advanced or metastatic; more further preferably is HR positive and HER-2 negative breast cancer which has or does not have PI3K/Akt/PTEN pathway alteration, and is refractory to standard treatment, locally advanced or metastatic.

5. The use according to claim 3 or 4, wherein the administration mode of the combined use is selected from simultaneous, concurrent, independent or sequential application; and the administration route of the combined use is selected from modes of oral, parenteral, rectal, pulmonary or topical administration, etc.

6. The use according to any one of claims 3-5, wherein the administration dose of the compound of Formula (A) is 0.1-1000 mg; preferably 20-500 mg; more preferably 100, 200, 300, 400 or 500 mg; further preferably 300, 400 or 500

mg;

the administration frequency of the compound of Formula (A) is once a day, twice a day, three times a day, once a week, once every two weeks, once every three weeks, or once a month; preferably once a day or twice a day;

preferably, the administration frequency of the compound of Formula (A) is once a day, twice a day, or three times a day, with continuous administration for 21 days followed by an interval of 1 to 2 weeks without administration of the compound; or continuous administration for 5 days or 4 days followed by an interval of 1 day to 3 days without administration of the compound;

further preferably, the administration frequency of the compound of Formula (A) is once a day or twice a day, with continuous administration for 21 days followed by an interval of 1 week without administration of the compound; or continuous administration for 5 days followed by an interval of 2 days without administration of the compound; or continuous administration for 4 days followed by an interval of 3 days without administration of the compound.

**7.** The use according to any one of claims 3-6, wherein the administration dose of fulvestrant is 0.1-800 mg; preferably 20-500 mg; more preferably 100, 200, 300, 400 or 500 mg; further preferably 300, 400 or 500 mg; and the administration frequency of fulvestrant is once a day, once a week, once every two weeks, once every three weeks, once every four weeks, once a month or once every two months; preferably once every two weeks or once every four weeks.

**8.** The use according to any one of claims 3-7, wherein one cycle is 28 days; the administration frequency of the compound of Formula (A) is continuous administration for 21 days, followed by an interval of 1 week between each administration cycle; the administration frequency of fulvestrant is once two weeks after the first administration in the first cycle, and then once on the first day of each subsequent cycle.

**9.** The use according to any one of claims 3-8, wherein the dose ratio of the compound of Formula (A) to fulvestrant is selected from 0.001-500:1, preferably 0.001-100:1.

**10.** The use according to any one of claims 1-9, wherein the pharmaceutically acceptable salt of the compound of Formula (A) is an organic acid salt or an inorganic acid salt; wherein the organic acid salt includes one or more of fumarate, mesylate, isethionate, $\alpha$-naphthalenesulfonate, p-toluenesulfonate, 1,2-ethanedisulfonate, oxalate, maleate, citrate, succinate, L-(+)-tartrate, hippurate, L-ascorbate, L-malate, benzoate or gentisate; the inorganic acid salt includes one or more of hydrochloride, sulfate or phosphate; preferably fumarate; more preferably monofumarate; further preferably monofumarate dihydrate.

**11.** A pharmaceutical composition or pharmaceutical combination, which comprises the compound of Formula (I) or pharmaceutically acceptable salt thereof, and fulvestrant, and one or more pharmaceutically acceptable carriers;

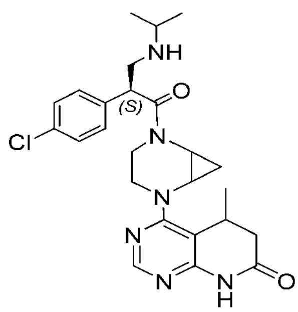

Formula (I) ;

preferably, the compound of Formula (I) is the compound of Formula (A) or the compound of Formula (B):

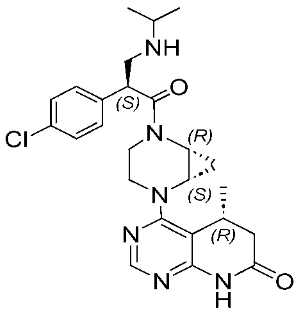 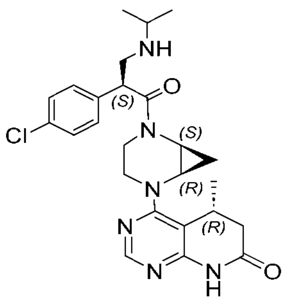

Formula (A) Formula (B) ;

preferably, the pharmaceutically acceptable salt of the compound of Formula (A) is an organic acid salt or an inorganic acid salt; wherein the organic acid salt includes one or more of fumarate, mesylate, isethionate, α-naphthalenesulfonate, p-toluenesulfonate, 1,2-ethanedisulfonate, oxalate, maleate, citrate, succinate, L-(+)-tartrate, hippurate, L-ascorbate, L-malate, benzoate or gentisate; the inorganic acid salt includes one or more of hydrochloride, sulfate or phosphate; preferably fumarate; more preferably monofumarate; further preferably monofumarate dihydrate.

**INTERNATIONAL SEARCH REPORT**

International application No.
**PCT/CN2024/099397**

**A. CLASSIFICATION OF SUBJECT MATTER**

A61K 31/519(2006.01)i; A61K 45/06(2006.01)i; A61P 35/00(2006.01)i; A61P 35/04(2006.01)i

According to International Patent Classification (IPC) or to both national classification and IPC

**B. FIELDS SEARCHED**

Minimum documentation searched (classification system followed by classification symbols)

IPC: A61K A61P

Documentation searched other than minimum documentation to the extent that such documents are included in the fields searched

Electronic data base consulted during the international search (name of data base and, where practicable, search terms used)

CNTXT; DWPI; ENTXTC; PUBMED; ELSEVIER; NCBI; VCN; Web of Science; STNext; CJFD; CNKI; 万方; WANFANG; 读秀; DUXIU: 正大天晴, 乳腺癌, 氟维司群, 激素受体, fulvestrant, Hormone Receptor, Estrogen Receptor, Progesterone Receptor, breast cancer, AKT, HER2, HER-2, HR, ER, PR, BT-474, MCF-7, ZR-75-1, T47D, CAMA-1, 2459489-66-6, 2741909-58-8, 2459489-67-7, 权利要求中的结构式检索, structural formula search in the claims

**C. DOCUMENTS CONSIDERED TO BE RELEVANT**

| Category* | Citation of document, with indication, where appropriate, of the relevant passages | Relevant to claim No. |
|---|---|---|
| X | WO 2020156437 A1 (NANJING CHIA TAI TIANQING PHARMACEUTICAL CO., LTD.) 06 August 2020 (2020-08-06) claims 11-16, and description, embodiment 15 | 1-10 |
| X | WO 2022017449 A1 (NANJING CHIA TAI TIANQING PHARMACEUTICAL CO., LTD.) 27 January 2022 (2022-01-27) claims 1-17 | 1-10 |
| X | WO 2022017446 A1 (NANJING CHIA TAI TIANQING PHARMACEUTICAL CO., LTD.) 27 January 2022 (2022-01-27) claims 1-15 | 1-10 |
| A | CN 114206345 A (F. HOFFMANN-LA ROCHE AG) 18 March 2022 (2022-03-18) claim 1 | 1-11 |
| A | WO 2020156437 A1 (NANJING CHIA TAI TIANQING PHARMACEUTICAL CO., LTD.) 06 August 2020 (2020-08-06) claims 11-16, and description, embodiment 15 | 11 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 September 2024** | **23 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/099397**

| Patent document cited in search report | Publication date (day/month/year) | Patent family member(s) | Publication date (day/month/year) |
|---|---|---|---|
| WO 2020156437 A1 | 06 August 2020 | CA 3127884 A1 | 06 August 2020 |
| | | KR 20210120054 A | 06 October 2021 |
| | | JP 2022517866 A | 10 March 2022 |
| | | EP 3919491 A1 | 08 December 2021 |
| | | EP 3919491 A4 | 19 October 2022 |
| | | US 2022144821 A1 | 12 May 2022 |
| | | AU 2020214258 A1 | 16 September 2021 |
| WO 2022017449 A1 | 27 January 2022 | JP 2023535702 A | 21 August 2023 |
| | | JP 7499942 B2 | 14 June 2024 |
| | | EP 4186902 A1 | 31 May 2023 |
| | | EP 4186902 A4 | 05 June 2024 |
| | | CA 3186568 A1 | 27 January 2022 |
| | | AU 2021314419 A1 | 23 March 2023 |
| | | AU 2021314419 A8 | 30 March 2023 |
| | | AU 2021314419 B2 | 07 December 2023 |
| | | US 2023286979 A1 | 14 September 2023 |
| WO 2022017446 A1 | 27 January 2022 | JP 2023534551 A | 09 August 2023 |
| | | CA 3186565 A1 | 27 January 2022 |
| | | US 2023321108 A1 | 12 October 2023 |
| CN 114206345 A | 18 March 2022 | EP 4013419 A1 | 22 June 2022 |
| | | JP 2022544485 A | 19 October 2022 |
| | | WO 2021030248 A1 | 18 February 2021 |
| | | US 2023330106 A1 | 19 October 2023 |
| | | US 2021046086 A1 | 18 February 2021 |
| | | US 11576919 B2 | 14 February 2023 |
| | | TW 202120096 A | 01 June 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023107072372
- WO 2020156437 A1
- WO 2022017448 A1